(19) Europäisches Patentamt
European Patent Office
Office européen des brevets

(11) Publication number: **0 255 964 B1**

(12) # EUROPEAN PATENT SPECIFICATION

(45) Date of publication of patent specification: **10.04.91** (51) Int. Cl.$^{5}$: **A61K 7/42**

(21) Application number: **87201116.8**

(22) Date of filing: **12.06.87**

(54) **Skin tanning agent.**

(30) Priority: **07.07.86 NL 8601767**

(43) Date of publication of application:
**17.02.88 Bulletin 88/07**

(45) Publication of the grant of the patent:
**10.04.91 Bulletin 91/15**

(84) Designated Contracting States:
**AT BE CH DE ES FR GB GR IT LI LU NL SE**

(56) References cited:
**EP-A- 0 007 120**
**CH-A- 642 537**
**DE-A- 2 722 725**

(73) Proprietor: **Chemisch Adviesbureau Drs. J.C.P. Schreuder B.V.**
**P.O. Box 430**
**NL-3740 AK Baarn(NL)**

(72) Inventor: **Schreuder, Johannes Carolus Petrus, Drs.**
**Van Reenenlaan 3**
**NL-3744 MD Baarn(NL)**

(74) Representative: **DIEHL GLAESER HILTL & PARTNER**
**Patentanwälte Flüggenstrasse 13**
**W-8000 München 19(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 255 964 B1

## Description

The invention is relating to a skin tanning composition and more particularly to a composition which may effect a relatively fast skin tanning after exposure to natural sunlight or exposure to the light from suntanning equipment such as sunbeds or solaria, whereas the skin is moreover not exposed to compounds, which may disturb the biological equilibrium of the skin.

Many compositions already have been proposed for skin tanning in the course of years.
Part of these proposed compositions contain as active main ingredient panthenol, which is applied in an acid, non aqueous phase, and are indeed effecting the desired skin tanning after exposure to natural or artificial sunlight, however, in the opinion of the modern consumer, too slowly.
For this reason compounds, which have to protect the skin temporarily against excessive interaction of harmful frequencies in the light spectrum, leading to burning, before the actual skin tanning process due to the natural production of melanoprotein (pigment) has taken place in a sufficient degree, have to be incorporated in these skin tanning agents.
These so called light filtering agents are still regarded undesired, due to a possible disturbance of the biological equilibrium of the skin and the toxicity of these compounds for humans in large concentrations, due to accumulation.

From EP-A-0 007 120 there was known a handwashing composition and a process for its preparation. The composition contains a continuous oil phase, mainly consisting of paraffinic oil fractions of 10 to 30 carbon atoms, a dispersed aqueous phase, a buffer, an emulsifying system, mainly consisting of mono- and diclycerides of the higher natural fatty acids and ethoxylated glycerine esterified by fatty acid, as well as glycerine and carraghenates. The composition is provided for cleaning skin which is exposed to an intensive filthiness caused by a large number of different works in the industry, such as works with paints, lacquers, glues, adhesives, printing inks, metal compositions, rusty metal parts for motor cars, cement and the like. The known composition has nothing to do with the tanning of skin.

An increasing need still exists for harmless, fast acting skin tanning compositions, wherein these before mentioned light filtering agents do not need to be applied.

Surprisingly, as a result of extensive and lengthy research, such a harmless, fast acting skin tanning composition could be developed, comprising at least the following characterizing ingredients:

- an oil fraction, consisting of straight or branched paraffinic oils, containing 10-30 carbon atoms in the chain, preferably 12-25 carbon atoms, most preferably 15-25 carbon atoms, in an amount of 5-50 % by weight and preferably from 10-40 % by weight, calculated on the weight of the total system.

These paraffinic oils have a boiling range of from 100-500 °C at atmospheric pressure and show a viscosity of at most 35 $mm^2/s$ (cSt) at 25 °C.

The paraffinic oils may be optionally mixed with esters of predominantly unsaturated higher natural fatty acids and of higher natural aliphatic alcohols, of at most 20 carbon atoms in the chain, such as oleyl oleate or oleyl decalate (e.g. Cetiol V®).

With the terms "higher natural fatty acids" and "higher natural alcohols" are meant fatty acids and alcohols which may be derived from products occurring in nature, such as animal or vegetable oils and fats, such as linseed oil, sunflower oil, rape oil, whale oil, castor oil, peanut oil, palm oil, olive oil, coconut oil, soybean oil, tung oil.

It appeared that for the most effective compositions, these esters have to be added to the paraffinic oil fractions in a amount up to 6% by weight and preferably 2-5 % by weight, based on the weight of the total composition.

- an emulsifying system, mainly consisting of

  a) mono- and/or diglycerides of higher unsaturated and/or saturated natural fatty acids, such as linoleic acid, oleic acid, linolenic acid, eleostearic acid, palmitic acid, lauric acid, or mixtures thereof (e.g. Tegomuls) in an amount of 1-10 % by weight and preferably 2-8 % by weight, calculated on the weight of the complete composition, and

  b) ethoxylated triglycerides, derived from fatty acids according

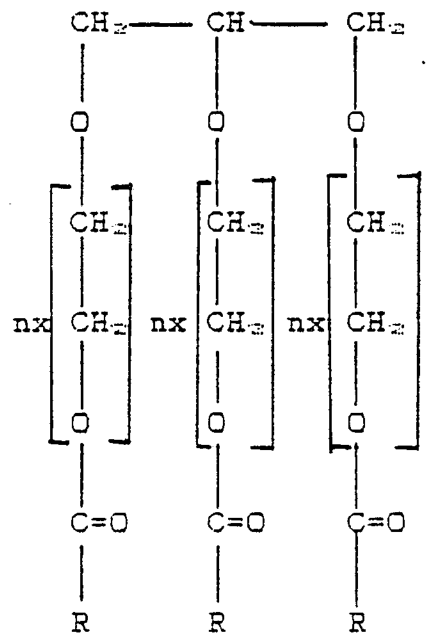

to the general formula: wherein n represents a number from 5-20 and preferably from 7-15, wherein R represents a saturated or unsaturated and preferably unsaturated fatty acid residue, derived from vegetable or animal oils, while R represents the same or different fatty acid residues in one molecule, but preferably the same (e.g. Tagat TO®).

It will be appreciated that the emulsifying system as described herein before, only will have to be applied in relatively small amounts of the decidedly indispensable, but with reference to skin affections undesired emulsifier, whereas on the other hand the present emulsifying system may be regarded as especially affable to the skin, which feature is connected with a relatively low hydrophilic-lipophylic balance value. The ratio between on the one hand the amount of the mono and diglycerides and on the other hand the ethoxylated triglycerides may vary, whereas the advantageous characteristics are maintained, from 10-100 parts of mono and diglycerides pro part of ethoxylated triglycerides and preferably 25 parts of mono and/or diglycerides pro part of ethoxylated triglycerides. The total amount of the emulsifying system, calculated on the weight of the total composition, may vary from 1-10 % by weight and preferably 2-8 % by weight, for the most optimal results.

- a stabilizer, consisting of montmorillonites, the free oxygen sites of which are occupied by quaternary groups (quaternary modified montmorillonites). Examples of such stabilizers which are preferably employed, are Bentone® or Propoloid® preparations, which are added in an amount of from 0.2-4 % by weight and preferably 0.5-2 % by weight, calculated on the weight of the total composition.

By addition of the beforementioned stabilizers, deposit of one or more of the composing ingredients is avoided in the relatively low viscous compositions, which are preferred for practical reasons.

- panthenol (provitamin B-5) in one of its pure optical isomeric forms or as racemic mixture, but preferably as D-panthenol, and tyrosine in one of its pure optical isomer forms or as racemic mixture, but preferably as L-tyrosine, or a compound from which the tyrosine easily may be formed in situ, such as e.g. esters from tyrosine or phenylalanine, which are known to be readily soluble in the aqueous phase.

It will be appreciated that it is generally known that phenylalanine can be easily converted into tyrosine under natural conditions.

With regard to the known bad solubility of tyrosine as such in an aqueous phase, the good properties of the presently proposed compositions could certainly not be expected or predicted by people skilled in the art.

Tyrosine is preferably added in the form of lower alkyl esters such as methyl, ethyl, (iso)propyl or (iso)-butyl ester or in the form of the stearyl ester or the benzyl ester, wherein the amino group optionally may be temporarily protected in a usual way and is preferably protected by HCl addition.

Additionally it was found that the aqueous phase, wherein the panthenol and the tyrosine ester ultimately have to be present, may have a pH in the range from 4-7 and more preferably in the range from 4-6, which may be obtained by addition of suitable alkalinity causing agent, such as sodium citrate and/or sodium hydroxide, especially in the case of protection of the amino group by HCl addition.

Amounts of e.g. sodium citrate and/or sodium hydroxide will vary, depending on the specific protecting groups in the starting tyroxine derivative, from 0,1-3 % by weight, calculated on the weight of the complete composition.

The molar ratio between tyrosine and panthenol should be in the range from 2:1 to 1:3 in order to reach the indicated attractive properties of the meant compositions aimed at, while the amounts of panthenol and tyrosine or tyrosine providing means, should be 1-5 % by weight (and preferably 2-4 % by weight) and 0.5-5 % by weight (preferably 1-4 % by weight) respectively, calculated on the weight of the complete composition.

- a preservative.
  Preferably different types of preservatives are applied for the ultimately formed continuous oily phase and the dispersed aqueous phase.
  For example esters of parahydroxy benzoic acid are used for the oily phase and preferably the methyl and/or the (iso)propyl ester and/or (iso)butyl ester is applied in an amount of 0.05-1 % by weight, calculated on the weight of the complete composition, and preferably in an amount of 0.2-0.4 % by weight.

Preferably mixtures of methyl, propyl and butyl-p-hydrobenzoate are used (e.g. Phenonip®).

It will be appreciated by a person skilled in the art that the beforementioned preservatives may be completely or partially replaced by other preservatives (e.g. Germall 115® or Hydroconserv®) in an amount of 0.05-1 % by weight and preferably 0.2-0.4 % by weight in the dispersed aqueous phase.

- water, ad 100 % by weight.

In addition to the beforementioned primary indispensable ingredients, one or more secondary ingredients also can be added to the final composition if desired, such as:

- vitamin E as such or preferably in the relatively stable acetate form, in an amount of 0.05-5 % by weight calculated on the weight of the complete composition and preferably in an amount of from 0.2-2 % by weight.
- glycerol, in an amount of 0.5-5.0 % by weight, calculated on the weight of the complete composition and preferably in an amount of from 1-3 % by weight, in order to form a moisture regulating system, together with the present panthenol and optionally co-added sodium lactate and/or possibly present alkali (to maintain the desired pH of from 4-7), e.g. sodium citrate.
  The total amount of alkali, such as sodium citrate, sodium hydoxide and optional sodium lactate will vary from 0,1-5 % by weight.
- a gelforming agent, such as carraghenate preferably consisting of a polysaccharide, bearing sulphonic acid residues, and preferably those of natural origin such as those derived from seaweeds. The sulphonic acid residues have optionally been converted into salts or esters of glycol, propylene glycol and glycerol (the so called modified carraghenates).

The beforementioned carraghenates cause a gel structure in the final complete composition, in the applied amounts of from 0.1-5 % by weight, calculated on the weight of the complete composition, and preferably 0.5-2 % by weight.

Such a complete composition shows a viscosity of 200-5000 mPa's (cP) at 25 °C, which is desired for an adequate application of the composition.

It will be appreciated by a person skilled in this specific art, that the carraghenate may be completely or particially replaced by alternative gel forming means, such as carboxy methyl cellulose, esterified by polyacrylic acid (e.g. Carbopol®) or hydroxy ethyl cellulose, in amounts which lead to viscosity values, situated in the beforementioned range.

- perfume, in an amount of from 0.1-0.5 % by weight, calculated on the weight of the complete composition.
- anti oxydants in an amount of from 0.01-3 % by weight, calculated on the weight of the complete composition.
- an alkanol, and more preferably ethanol (96 %) in an amount of from 0.1-1.0 % by weight and preferably 0.3-0.6 % by weight, calculated on the weight of the complete composition.
  The alkanol may be added to reach a fast gelation of the quaternary modified montmorillonites.

The compositions according to the present invention are characterized by a relatively low viscosity and high stability, which guarantees an easy application without a "greasy" feeling or stickyness and stains in clothes due to a fast penetration into the skin tissue, and give the skin the desired tanning by means of natural pigment formation in the skin after a relatively short period of exposure to light.

The composition according to the present invention may be prepared by a process, which should be regarded as another aspect of the invention.

This process is characterized by a specific sequence of addition and the dosing rate of the respective

beforementioned ingredients and by the temperature and stirring speeds at which this addition c.q. dosing takes place.

The first step of this process comprises the preparation of the complete continuous oily phase, composed of the oil fractions, the esters of the unsaturated fatty acids and alcohols, emulsifying system, stabilisers, preservatives and alkanol, e.g. ethanol, whereas the dispersed aqueous phase is composed of water, glycerol, panthenol, tyrosine or tyrosine providing compound, desired alkali and carraghenate.
Vitamin E and the possible perfume may be added to the prepared emulsion.

The compositions according to the present invention are preferably prepared by composing both phases and mixing them together at a temperature of at most $30\,^{\circ}C$, followed by additional stirring, after the possible addition of Vitamin E and perfume, until an average particle size of the dispersed aquous phase of at most 5 $\mu$, and preferably smaller than 3 $\mu$, is reached.

It will be appreciated by a person skilled in the art that the application of the before described compositions, i.e. the treatment of the skin with the before described compositions, forms another aspect of the inventiom.
Such an application comprises a method, being usual for such compositions, characterized by application and spreading evenly on the skin area involved of an amount of 20-100 $ml/m^2$ skin area, if necessary after thorough cleaning of the skin with water an soap or an alcoholic solution, preceeding the exposure of the skin to natural or artificial sunlight.
This treatment should preferably be repeated 2-3 times a day.

The invention is illustrated on basis of the subsequent examples, however without restricting the scope of it thereto:

EXAMPLE 1

Under stirring the following ingredients are combined:

| | | |
|---|---|---|
| Parrafinic oil I (Shell Ondina 15®, boiling range 295-390°C) | 130 g | |
| Paraffinic oil II (Shell Ondina 68®, boiling range 290-500°C) | 30 g | |
| Mono and/or diglycerides (Tegomuls®) | 25 g | |
| Oleyl decalate (Cetiol V®) | 30 g | oily phase |
| Ethoxylated triglycerides (Tagat TO®) | 1 g | |
| Preservative I (Phenonip®) | 2 g | |
| followed by addition of quaternary modified montmorillonites (Bentone 38®) | 6 g | |
| and gelation by addition of ethanol 96 % | 3 g | |

The mixture is cooled to atmost 30 ° C
In water (687 g) are subsequently dissolved:

| | | |
|---|---|---|
| Glycerol | 20 g | aqueous phase |
| Phenyl alanine | 20 g | |
| Sodium citrate | 10 g | |
| D-panthenol | 16 g | |
| Preservative II (Hydroconserv®) | 3 g | |
| Carraghenate | 5 g | |

The obtained aqueous phase is subsequently mixed with the oily phase and homogenized after addition of vitamin E-acetate (D-isomer) (10 g) and perfume (2 g). The obtained water in oil emulsion is further homogenized until an average particle size < 3 μ is reached.

EXAMPLE 2

In the same way as described under example 1, a composition was prepared from the following ingredients:

| | | | |
|---|---|---|---|
| Paraffinic oil I | 250 g | | oily phase |
| Paraffinic oil II | 50 g | | |
| Mono and/or diglycerides | 40 g | | |
| Ethoxylated triglycerides | 2 g | | |
| Preservative I | 2 g | | |
| Quaternary modified montmorillonites (Bentone 27®) | 6 g | | |
| Ethanol | 3 g | | |

and

| | | | |
|---|---|---|---|
| Water | 580 g | | aqueous phase |
| Glycerol | 15 g | | |
| L-tyrosine ethylate HCl | 10 g | | |
| Sodium Hydroxide | 1 g | | |
| D-panthenol | 10 g | | |
| Sodium lactate | 10 g | | |
| Preservative I | 3 g | | |
| Carraghenate | 8 g | | |

and

| | |
|---|---|
| Vitamin E-acetate (D-isomer) | 8 g |
| Perfume | 2 g |

EXAMPLE 3

In the same way as described under example 1, a composition was prepared from the following ingredients:

| | | |
|---|---|---|
| Paraffinic oil I | 150 g | oily phase |
| Paraffinic oil II | 40 g | |
| Mono and/or diglycerides | 30 g | |
| Ethoxylated triglycerides | 1 g | |
| Oleyl oleate | 20 g | |
| Preservative I | 2 g | |
| Quaternary modified montmorillonites (Bentone 38®) | 6 g | |
| Ethanol | 2 g | |

and

| | | |
|---|---|---|
| Water | 659 g | aqueous phase |
| Glycerol | 15 g | |
| L-tyrosine isobutylate | 30 g | |
| Sodium citrate | 10 g | |
| D-panthenol | 20 g | |
| Preservative III (Euxyl 100®) | 3 g | |
| and | | |
| Vitamin E-acetate (D-isomer) | 10 g | |
| Perfume | 2 g | |

EXAMPLE 4

In the same way as described under example 1, a composition was prepared from the following ingredients:

| | | | |
|---|---|---|---|
| Paraffinic oil I | 140 | g | oily phase |
| Paraffinic oil II | 25 | g | |
| Mono and/or diglycerides | 15 | g | |
| Oleyl decalate | 26 | g | |
| Ethoxylated triglycerides | 0.5 | g | |
| Preservative I | 3 | g | |
| Quaternary modified montmorillonites (Bentone 27®) | 7 | g | |
| Ethanol | 3 | g | |

and

| | | | |
|---|---|---|---|
| Water | 692.5 | g | aqueous phase |
| Glycerol | 20 | g | |
| L-tyrosine ethylate HCl | 15 | g | |
| Sodium hydroxide | 10 | g | |
| D-panthenol | 15 | g | |
| Sodium lactate | 4 | g | |
| Sodium citrate | 3 | g | |
| Preservative I | 3 | g | |
| Carraghenate | 8 | g | |

and

| | | |
|---|---|---|
| Vitamin E-acetate (D-isomer) | 7 | g |
| Perfume | 3 | g |

EXAMPLE 5

In the same way as described under example 1, a composition was prepared from the following ingredients:

| | | | |
|---|---|---|---|
| Paraffinic oil I | 200 g | oily phase | |
| Paraffinic oil II | 50 g | | |
| Mono and/or diglycerides | 40 g | | |
| Ethoxylated triglycerides | 2 g | | |
| Preservative I | 2 g | | |
| Quaternary modified montmorillonites (Bentone 27®) | 6 g | | |
| Isopropanol | 2 g | | |

and

| | | |
|---|---|---|
| Water | 613 g | aqueous phase |
| Glycerol | 15 g | |
| D,L-tyrosine isobutylate | 25 g | |
| D,L-Panthenol | 20 g | |
| Sodium citrate | 10 g | |
| Preservative III | 3 g | |
| Vitamin E-acetate (D,L) | 10 g | |
| Perfume | 2 g | |

The beforementioned compositions caused a fast, intensive natural skin tanning within some hours exposure after the application in the usual way.

## Claims

1. Process for the preparation of a skin tanning composition, characterized in that an oily phase is prepared from at least the following ingredients:
   - an oil fraction, comprising straight or branched paraffinic oils, having 10-30 carbon atoms in the chain, in an amount of 5-50 % by weight, calculated on the weight of the complete composition, having a boiling range between 100 and 500 °C at atmospheric pressure and a viscosity of at most 35 $mm^2/s$ (cSt) at 25 °C, wherein the paraffinic oils may optionally be mixed with esters of optionally unsaturated higher natural fatty acids and of optionally unsaturated higher natural aliphatic alcohols, of at most 20 carbon atoms in the chain, in an amount of up to 6 % by weight, calculated on the weight of the complete composition,
   - an emulsifying system, mainly comprising
     a) mono and/or diglycerides of higher unsaturated and/or saturated natural fatty acids, in an amount of 1-10 % by weight, calculated on the weight of the complete composition, and
     b) ethoxylated triglycerides, esterified with fatty acids according to the general formula

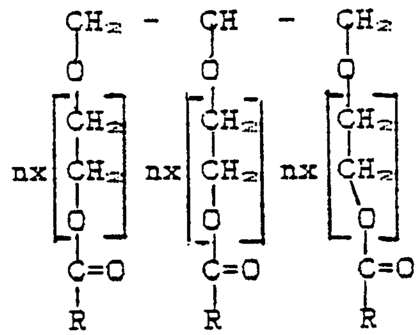

wherein n represents an integer of from 5-20 and R represents a saturated or unsaturated fatty acid residue, derived from vegetable or animal oils, wherein R represents the same or different fatty acid residues in one molecule, whereby the ratio between the amount of mono- and/or diglycerides on the one hand and of ethoxylated triglycerides on the other hand may vary from 10-100 parts mono- and/or diglycerides pro part ethoxylated triglycerides, wherein the complete amount of the emulsifying system, calculated on the weight of the complete composition, may vary from 1-10 % by weight,.

- a stabilizer, consisting of montmorillonites, the free oxygen sites of which are occupied by quaternary groups, in an amount of from 0.2-4 % by weight, calculated on the weight of the complete composition,
- a preservative being the same or different for the formed continuous oily phase and the dispersed aqueous phase, in an amount of from 0.05-1 % by weight, calculated on the weight of the complete composition, under stirring and heating until gelation, after optional addition of alkanol in an amount of from 0.1-1.0 % by weight, calculated on the weight of the complete composition, followed by cooling to at most 30° C and addition of an aqueous phase, prepared from at least the following ingredients:
- panthenol, and tyrosine, or a compound from which tyrosine may be easily formed in situ in the aqueous phase, in a mutual molar ratio of tyrosine:panthenol between 2:1 and 1:3, wherein the amounts of panthenol and tyrosine or tyrosine providing means may be from 1-5 % by weight and from 0.5-5 % by weight, respectively, calculated on the weight of the complete composition,
- alkalinity causing agent to adjust pH of the aqueous phase to be in the range from 4-7,
- water ad 100 % by weight, followed by stirring and homogenising until an average particle size < 3 μ is reached.

**2.** Process according to claim 1, characterized in that the following ingredients may be additionally added to the aqueous phase:
- glycerol, in an amount of 0.5-5.0 % by weight, calculated an the weight of the complete composition
- sodium lactate and/or sodium citrate in an amount of 0.1-5 % by weight,
- gel forming agent, in an amount of from 0.1-5 % by weight.

**3.** Process according to claims 1 and 2, characterized in that additional ingredients are added, consisting of
- vitamin E, in an amount of from 0.05-5 % by weight, and/or,
- perfume, in an amount of 0.1-0.5 % by weight,
- anti-oxydant, in an amount of 0.01-3 % by weight, calculated on the weight of the complete composition.

**4.** Process according to claim 1, characterized in that a composition is prepared, containing an oil fraction in an amount of 10-40 % by weight.

**5.** Process according to claim 1, characterized in that a composition is prepared, containing an oil fraction mixed with oleyl oleate or oleyl decalate in an amount of 2-5 % by weight, calculated on the weight of the complete composition.

6. Process according to claim 1, characterized in that the emulsifying system is composed of ethoxylated triglycerides, derived from linoleic acid, oleic acid, linolenic acid or mixtures thereof, mixed with mono and/or diglycerides derived from linoleic acid, linolenic acid, oleic acid, palmitic acid, lauric acid, miristic acid, stearic acid, eleostearic acid and mixtures thereof.

7. Process according to claim 1, characterized in that the ratio between mono and/or diglycerides and ethoxylated triglycerides is 25 parts mono and/or diglycerides pro part ethoxylated triglycerides.

8. Process according to claim 1, characterized in that as preservative in the oily phase one or more esters of p-hydroxybenzoic acid are used in an amount of 0.2-0.4 % by weight.

9. Process according to any of claims 1 to 8, characterized in that there is used an oil fraction comprising straight or branched paraffinic oils having 12 - 25 carbon atoms in the chain.

10. Process according to any of claims 1 to 9, characterized in that there is used an oil fraction comprising straight or branched paraffinic oils in an amount of 10 - 40 % by weight.

11. Process according to any of claims 1 to 10, characterized in that in the emulsifying system there is used an esterified ethoxylated triglyceride of the general formula as indicated in claim 1 wherein n represents an integer of 7 - 15.

12. Process according to any of claims 1 to 11 characterized in that in the emulsifying system there is used an esterified ethoxylated triglyceride of the general formula as indicated in claim 1 wherein R represents an unsaturated fatty acid residue.

13. Process according to any of claims 1 to 12, characterized in that in the emulsifying system there is used an esterified ethoxylated triglyceride of the general formula as indicated in claim 1 wherein the residues R represent the same fatty acid residues in one molecule.

14. Process according to any of claims 1 to 13, characterized in that there is used panthenol in form of D-panthenol.

15. Process according to any of claims 1 to 14, characterized in that there is used tyrosine in form of L-tyrosine.

16. Process according to any of claims 1 to 15, characterized in that there is used the alkalinity causing agent to adjust the pH of the aqueous phase in the range of 4 - 6.

17. Process according to any of claims 1 to 16, characterized in that there is used the alkalinity causing agent in form of sodium citrate and/or sodium hydroxide.

18. Process according to claim 2, characterized in that there is used glycerol in an amount of 1 - 3 % by weight, calculated on the weight of the complete composition.

Claims for the following Contracting States: BE CH DE FR GB IT LI LU NL SE:

1. Skin tanning composition, characterized in that it is at least comprising the following ingredients:
   - an oil fraction, comprising straight or branched paraffinic oils, having 10-30 carbon atoms in the chain in an amount of 5-50 % by weight, calculated on the weight of the complete composition, having a boiling range between 100 and 500 °C at atmospheric pressure and a viscosity of at most 35 $mm^2/s$ (cSt) at 35 °C, wherein the paraffinic oils may optionally be mixed with esters of optionally unsaturated higher natural fatty acids and of optionally unsaturated higher natural aliphatic alcohols of at most 20 carbon atoms in the chain, in an amount of up to 6 % by weight, calculated on the weight of the complete composition.
   - an emulsifying system, mainly comprising:
     a) mono and/or diglycerides of higher unsaturated and/or saturated natural fatty acids, in an amount of 1-10 % by weight, calculated on the weight of the complete composition, and
     b) ethoxylated triglycerides, esterified with fatty acids according to the general formula

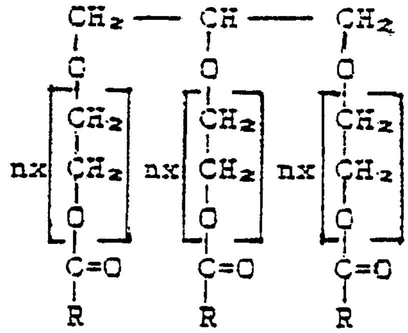

wherein n represents an integer of from 5 -20 and R represents a saturated or unsaturated fatty acid residue, derived from vegetable or animal oils, wherein R represents the same or different fatty acid residues in one molecule, whereby the ratio between the amount of mono- and/or diglycerides on the one hand and of the ethoxylated triglycerides on the other hand may vary from 10-100 parts mono- and/or diglycerides pro part ethoxylated triglycerides, wherein the complete amount of the emulsifying system. calculated on the weight of the complete composition, may vary from 1-10 % by weight,

- panthenol and tyrosine or a compound from which tyrosine may be easily formed in situ in the aqueous phase, in a mutual molar ratio of tyrosine : panthenol between 2:1 and 1:3, wherein the amounts of panthenol and tyrosine or tyrosine providing means may be from 1-5 % by weight and from 0.5-5 % by weight, respectively, calculated on the weight of the complete composition,
- alkalinity causing agent to adjust the pH of the aqueous phase to be in the range from 4-7,
- a stabilizer, consisting of montmorillonites, the free oxygen sites of which are occupied by quaternary groups, in a amount of from 0.2-4 % by weight, calculated on the weight of the complete composition,
- a preservative, being the same or different for the formed continuous oily phase and the dispersed aqueous phase, in an amount of from 0.05-1 % by weight, calculated on the weight of the complete composition,
- water ad 100% by weight.

2. Composition according to claim 1, characterized in that it contains in addition to the indicated ingredients, one or more of the following ingredients:
   - vitamin E, in an amount of 0.05-5% by weight,
   - glycerol, in an amount of 0.5-5.0 % by weight,
   - alkanol in an amount of 0.1-1.0% by weight,
   - sodium lactate, in an amount of 0.1-5% by weight,
   - gel forming agent, in an amount or 0.1-5% by weight,
   - perfume, in an amount of 0.1-0.5% by weight,
   - anti oxydants, in an amount of 0.01-3% by weight, calculated on the weight of the complete composition.

3. Composition according to claim 1, characterized in that it contains an oil fraction in an amount of 10-40% by weight.

4. Composition according to claim 1, characterized in that it comprises an oil fraction mixed with oleyl oleate or oleyl decalate in an amount 0f 2-5% by weight, calculated on the weight of the complete composition.

5. Composition according to claim 1, characterized in that the emulsifying system is composed of ethoxylated triglycerides, derived from linoleic acid, oleic acid, linolenic acid or mixtures thereof, mixed with mono and/or diglycerides derived from linoleic acid, linolenic acid, oleic acid, palmitic acid, lauric acid, miristic acid, stearic acid, eleostearic acid and mixtures thereof.

6. Composition according to claim 1, characterized in that the ratio between mono- and/or diglycerides and ethoxylated triglycerides is 25 parts mono-and/or diglycerides pro part ethoxylated triglycerides.

7. Composition according to claim 1, characterized in that as preservative in the oily phase one or more esters of p-hydroxybenzoic acid are used in an amount of 0.2-0.4% by weight.

8. Composition according to any of claims 1 to 7, characterized in that the oil fraction comprises straight or branched paraffinic oils having 12 - 25 carbon atoms in the chain.

9. Composition according to any of claims 1 to 8, characterized in that the oil fraction comprises straight or branched paraffinic oils in an amount of 10 - 40 % by weight.

10. Composition according to any of claims 1 to 9, characterized in that in the general formula of the esterified ethoxylated triglycerides of the emulsifying system, as indicated in claim 1, n represents an integer of 7 - 15.

11. Composition according to any of claims 1 to 10, characterized in that in the general formula of the esterified ethoxylated triglycerides of the emulsifying system, as indicated in claim 1, R represents an unsaturated fatty acid residue.

12. Composition according to any of claims 1 to 11, characterized in that in the general formula of the esterified ethoxylated triglycerides of the emulsifying system, as indicated in claim 1, the residues R represent the same fatty acid residues in one molecule.

13. Composition according to any of claims 1 to 12, characterized in that it comprises panthenol in form of D-panthenol.

14. Composition according to any of claims 1 to 13, characterized in that it comprises tyrosine in form of L-tyrosine.

15. Composition according to any of claims 1 to 14, characterized in that it comprises the alkalinity causing agent to adjust the pH of the aqueous phase in the range of 4 - 6.

16. Composition according to any of claims 1 to 15, characterized in that it comprises the alkalinity causing agent in form of sodium citrate and/or sodium hydroxide.

17. Composition according to claim 2, characterized in that it contains sodium lactate in an amount of 0.5 - 2 % by weight.

18. Process for the preparation of a composition according to any of claims 1-17 characterized in that an oily phase is prepared by addition of the emulsifying system. preservative and stabilizer to the oily components under stirring and heating until gelation after addition of alcohol, followed by cooling to at most 30 °C and addition of the aqueous phase, composed of water, preservative, glycerol, panthenol, tyrosine or tyrosine providing compound alkalinity causing agent, and carraghenate.

19. Process for the preparation of a skin tanning composition, characterized in that an oily phase is prepared from at least the following ingredients:
   - an oil fraction as defined in any of claims 1,8 or 9,
   - an emulsifying system as defined in any of claims 1, 10, 11 or 12,
   - a stabilizer as defined in claim 1,
   - a preservative as defined in claim 1, under stirring and heating until gelation. After optional addition of alkanol in an amount of from 0.1-1.0% by weight, calculated on the weight of the complete composition, followed by cooling to at most 30 °C and addition of an aqueous phase, prepared from at least the following ingredients:
   - panthenol, and tyrosine, or a compound from which tyrosine may be easily formed in situ in the aqueous phase, in a mutual molar ratio of tyrosine: panthenol between 2:1 and 1:3, wherein the amounts of panthenol and tyrosine or tyrosine providing means may be from 1-5% by weight and from 0.5-5% by weight, respectively, calculated on the weight of the complete composition,
   - alkalinity causing agent to adjust the pH of the aqueous phase to be in the range from 4-7,
   - water ad 100 % by weight, followed by stirring and homogenising until an average particle size <3 $\mu$ is reached.

**20.** Process according to claim 19, characterised in that there is used panthenol in form of D-panthenol, or tyrosine in form of L-tyrosine, or the alkalinity causing agent to adjust the pH of the aqueous phase to be in the range of 4 - 6, or the alkalinity causing agent in form of sodium citrate and/or sodium hydroxide.

**21.** Process according to claim 20, characterized in that the following ingredients may be additionally added to the aqueous phase:

- glyerol, in an amount of 0.5-5.0 % by weight, calculated on the weight of the complete composition
- sodium lactate and/or sodium citrate in an amount of 0.1-5 % by weight,
- gel forming agent, in an amount of from 0.1-5 % by weight.

**22.** Process according to claim 21, characterized in that glycerol is used in an amount of 1 - 3 % by weight, calculated on the weight of the complete composition.

**23.** Process according to any of claims 19 to 22, characterised in that additional ingredients are added, consisting of

- vitamin E, in an amount of from 0.05-5 % by weight, and/or,
- perfume, in an amount of 0.1-0.5 % by weight,
- anti-oxydant, in an amount of 0.01-3 % by weight, calculated on the weight of the complete composition.

**24.** Process according to claim 19, characterised in that a composition is prepared, Containing an oil fraction in an amount of 10-40 % by weight.

**25.** Process according to claim 19, characterised in that a composition is prepared, containing an oil fraction mixed with oleyl oleate or oleyl decalate in an amount of 2-5 % by weight, calculated on the weight of the complete composition.

**26.** Process according to claim 19, characterised in that the emulsifying system is composed of ethoxylated triglycerides, derived from linoleic acid, oleic acid, linolenic acid or mixtures thereof, mixed with mono and/or diglycerides derived from linoleic acid, linolenic acid, oleic acid, palmitic acid, lauric acid, miristic acid. stearic acid, eleostearic acid and mixtures thereof.

**27.** Process according to claim 19, characterised in that the ratio between mono and/or diglycerides and ethoxylated triglycerides is 25 parts mono- and/or diglycerides pro part ethoxylated triglycerides.

**28.** Process according to claim 19, characterized in that as preservative in the oily phase one or more esters of p-hydroxybenzoic acid are used in an amount of 0.2-0.4 % by weight.

**Revendications**

**1.** Composition de bronzage de la peau, caractérisée en ce qu'elle comprend au moins les ingrédients suivants :

- une fraction huileuse comprenant des huiles paraffiniques linéaires ou ramifiées, ayant 10 à 30 atomes de carbone dans la chaine, en une quantité de 5 à 50% en poids, sur la base du poids de la composition complète, ayant une plage d'ébullition comprise entre 100 et 500°C à la pression atmosphérique et une Viscosité d'au moins 35 $mm^2/s$ (cSt) à 25°C,dans laquelle les huiles paraffiniques peuvent facultativement être mélangées avec des esters d'acides gras naturels de rang élevé facultativement insaturés et d'alcools aliphatiques naturels de degré élevé facultativement insaturés d'au moins 20 atomes de carbone dans la chaîne, en une quantité pouvant atteindre 6% en poids, sur la base du poids de la composition complète;
- un système émulsifiant comprenant principalement :

  a) des mono- et/ou des diglycérides d'acides gras naturels de degré élevé insaturés et/ou saturés, en une quantité de 1 à 10% en poids, sur la base du poids de la composition complète, et

  b) des triglycérides éthoxylés, estérifiés avec des acides gras selon la formule générale :

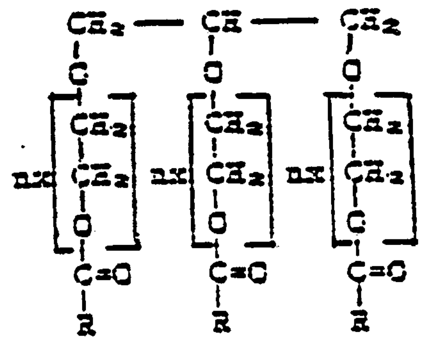

dans laquelle n est un entier de 5 à 20 et R reprèsente un résidu d'acides gras saturés ou insaturés, dérivés d'huiles végétales ou animales, dans laquelle R représente des résidus d'acides gras identiques ou différents dans une molécule, d'où il résulte que le rapport entre la quantité des monoet/ou diglycérides d'une part, et des triglycérides éthoxylés d'autre part peut varier de 10 à 100 parties de mono- et/ou de diglycérides pour une partie de triglycérides éthoxylés, la quantité complète du système émulsifiant, sur la base du poids de la composition complète, pouvant varier de 1 à 10% en poids;

- du panthénol et de la tyrosine ou un composé dont peut être aisément être formée in situ la tyrosine dans la phase aqueuse, en un rapport molaire mutuel de tyrosine : panthénol compris entre 2 : 1 et 1 : 3, les quantités de panthénol et de tyrosine ou du composé procurant la tyrosine pouvant être de 1 à 5% en poids et de 0,5 à 5% en poids respectivement, sur la base du poids de la composition complète;
- un agent causant une alcalinité de la phase aqueuse pour amener le pH entre 4 et 7;
- un stabilisant, constitué par des montmorillonites, dont les sites d'oxygène libre sont occupés par des groupes quaternaires, en une quantité de 0,2 à 4% en poids, sur la base du poids de la composition complète;
- un conservateur, identique ou différent pour la phase huileuse continue et pour la phase aqueuse dispersée, formées, en une quantité de 0,05 à 1% en poids, sur la base du poids de la composition complète;
- eau pour compléter à 100% en poids.

**2.** Composition selon la revendication 1, caractérisée en ce qu'elle contient, en plus des ingrédients indiqués,un ou plusieurs des ingrédients suivants :

- vitamine E, en une quantité de 0,05 à 5% en poids,
- glycérol, en une quantité de 0,5 à 5,0% en poids,
- alcanol, en une quantité de 0,1 à 1% en poids,
- lactate de sodium, en une quantité de 0,1 à 5% en poids,
- agent formateur de gel, en une quantité de 0,1 à 5% en poids,
- parfum, en une quantité de 0,1 à 0,5% en poids,
- anti-oxydants, en une quantité de 0,01 à 3% en poids, sur la base du poids de la composition complète.

**3.** Composition selon la revendication 1, caractérisée en ce qu'elle contient une fraction huileuse en une quantité de 10 à 40% en poids.

**4.** Composition selon la revendication 1, caractérisée en ce qu'elle comprend une fraction huileuse mélangée avec de l'oléate d'oléyle ou du décalate d'oléyle en une quantité de 2 à 5% en poids, sur la base du poids de la composition complète.

**5.** Composition selon la revendication 1, caractérisée en ce que le système émulsifiant comprend des triglycérides éthoxylés, dérivés de l'acide linoléique, l'acide oléique, l'acide linolénique ou de leurs mélanges, mélangés avec des mono- et/ou des diglycérides dérivés de l'acide linoléique, l'acide linolénique, l'acide oléique, l'acide palmitique, l'acide laurique, l'acide myristique, l'acide stéarique, l'acide éléostéarique et leurs mélanges.

**6.** Composition selon la revendication l' caractérisée en ce que le rapport entre les mono- et/ou les diglycérides et les triglycérides éthoxylés est de 25 parties de monoet/ou de diglycérides pour 1 partie de triglycérides éthoxylés.

**7.** Composition selon la revendication 1, caractérisée en ce qu'on utilise en tant que conservateur dans la phase huileuse un ou plusieurs esters d'acide p-hydroxybenzoîque en une quantité de 0,2 à 0,4% en poids.

**8.** Composition selon l'une des revendications 1 à 7, caractérisée en ce que la fraction huileuse comprend des huiles paraffiniques linéaires ou ramifiées ayant 12 à 25 atomes de carbone dans la chaine.

**9.** Composition selon l'une des revendications 1 à 8, caractérisée en ce que la fraction huileuse comprend des huiles paraffiniques linéaires ou ramifiées en une quantité de 10 à 40% en poids.

**10.** Composition selon l'une des revendications 1 à 9, caractérisée en ce que, dans la formule générale des triglycérides éthoxylés estérifiés du système émulsifiant, comme indiqué dans la revendication 1, n représente un entier de 7 à 15.

**11.** Composition selon l'une des revendications 1 à 10, caractérisée en ce que, dans la formule générale des triglycérides éthoxylés estérifiés du système émulsifiant, comme indiqué dans la revendication 1, R représente un résidu d'acides gras insaturés.

**12.** Composition selon l'une des revendications 1 à 11, caractérisée en ce que, dans la formule générale des triglycérides éthoxylés estérifiés du système émulsifiant, comme indiqué dans la revendication 1, les résidus R représentent les résidus du même acide gras dans une molécule.

**13.** Composition selon l'une des revendications 1 à 12, caractérisée en ce qu'elle comprend du panthénol sous la forme de D-panthénol.

**14.** Composition selon l'une des revendications 1 à 13, caractérisée en ce qu'elle comprend de la tyrosine sous la forme de L-tyrosine.

**15.** Composition selon l'une des revendications 1 0 14, caractérisée en ce qu'elle comprend l'agent causant l'alcalinité pour ajuster le pH de la phase aqueuse dans la plage de 4 à 6.

**16.** Composition selon l'une des revendications 1 à 15, caractérisée en ce qu'elle comprend l'agent causant l'alcalinité sous la forme de citrate de sodium et/ou d'hydroxyde de sodium.

**17.** Composition selon la revendication 2, caractérisée en ce qu'elle contient du lactate de sodium en une quantité de 0,5 à 2% en poids.

**18.** Procédé pour la préparation d'une composition selon l'une des revendications 1 à 17, caractérisé en ce qu'on prépare une phase huileuse par addition du système émulsifiant, du conservateur et du stabilisant aux composants huileux en agitant et en chauffant jusqu 'à gélification après addition d'un alcool, après quoi on refroidit à au plus 30 ° C et on ajoute la phase aqueuse, comprenant de l'eau, un conservateur, du glycérol, du panthénol, de la tyrosine ou un composé procurant la tyrosine, un agent causant l'alcalinité, si nécessaire, et un carraghénate.

**19.** Procédé pour préparer une composition de bronzage de la peau, caractérisé en ce qu'on prépare une phase huileuse à partir d'au moins les ingrédients suivants :

- une fraction huileuse comme définie dans l' une des revendications 1, 8 ou 9,
- un système émulsifiant, comme défini dans l'une des revendications 1, 10, 11 ou 12,
- un stabilisant, comme défini dans la revendication 1,
- un conservateur, comme défini dans la revendication 1, en agitant et en chauffant jusqu' à gélification, après addition facultativbe d'alcanol en une quantité de 0,1 à 1% en poids, sur la base du poids de la composition complète, après quoi on refroidit à au plus 30°C et on ajoute une phase aqueuse, comprenant au moins les ingrédients suivants:
- panthénol et tyrosine ou un composé dont on peut aisément former de la tyrosine in situ dans la

phase aqueuse, en un rapport molaire mutuel de tyrosine:panthénol compris entre 2:1 et 1:3, les quantités de panthénol et de tyrosine ou du composé procurant la tyrosine pouvant aller de 1 à 5% en poids et de 0,5 à 5% en poids respectivement, sur la base du poids de la composition complète,

- un agent causant l' alcalinité pour ajuster le pH de la phase aqueuse dans la plage de 4 à 7,
- de l'eau pour compléter à 100% en poids, après quoi on ajoute et on homogénéise jusqu'à obtencion d'une dimension moyenne de particules inférieure à 3 $\mu$m.

**20.** Procédé selon la revendication 19, caractérisé en ce qu'on utilise le panthénol sous la forme de D-panthénol, ou la tyrosine sous la forme de L-tyrosine, ou l'agent causant l' alcalinité pour ajuster le pH de la phase aqueuse dans la plage de 4 à 6, ou l'agent causant l'alcalinité sous la forme de citrate de sodium et/ou d'hydroxyde de sodium.

**21.** Procédé selon la revendication 20, caractérisé en ce qu'on peut en outre ajouter les ingrédients suivants à la phase aqueuse :

- glycérol, en une quantité de 0,5 à 5,0% en poids, sur la base du poids de la composition complète,
- du lactate de sodium et/ou du citrate de sodium en une quantité de 0,1 à 5% en poids,
- un agent formateur de gel en une quantité de 0,1 à 5% en poids.

**22.** Procédé selon la revendication 21, caractérisé en ce qu'on utilise le glycérol en une quantité de 1 à 3% en poids, sur la base du poids de la composition complète.

**23.** Procédé selon l'une des revendications 19 à 22, caractérisé en ce qu'on ajoute des ingrédients additionnels, à savoir :

- vitamine E, en une quantité de 0,05 à 5% en poids, et/ou
- parfum, en une quantité de 0,1 à 0,5% en poids,
- anti-oxydant, en une quantité de 0,01 à 3% en poids, sur la base du poids de la composition complète.

**24.** Procédé selon la revendication 19, caractérisé en ce qu'on prépare une composition contenant une fraction huileuse en une quantité de 10 à 40% en poids.

**25.** Procédé selon la revendication 19, caractérisé en ce qu'on prépare une composition, contenant une fraction huileuse mélangée avec de l'oléate d'oléyle ou du décalate d'oléyle en une quantité de 2 à 5% en poids, sur la base du poids de la composition complète.

**26.** Procédé selon la revendication 19, ca'atérsé en ce que le système émulsifiant comprend des triglycérides éthoxylés, dérivés de l'acide linoléique, l'acide oléique, l'acide linolénique ou leurs mélanges, mélangés avec des mono- et/ou des diglycérides dérivés de l'acide linoléique, l'acide linolénique, l'acide oléique, l'acide palmitique, l'acide laurique, l'acide myristique, l'acide stéarique, l'acide éléostéarique et leurs mélanges.

**27.** Procédé selon la revendication 19, caractérisé en ce que le rapport entre les mono- et/ou les diglycérides et les triglycérides éthoxylés est 25 parties de mono- et/ou de diglycérides pour 1 partie de triglycérides éthoxylés.

**28.** Procédé selon la revendication 19, caractérisé en ce qu'on utilise, comme conservateur dans la phase huileuse, un ou plusieurs esters d'acide p-hydroxybenzoîque en une quantité de 0,2 à 0,4% en poids.

Revendications pour les Etats contractants suivants: AT ES GR.

**1.** Procédé pour la préparation d'une composition de bronzage de la peau, caractérisé en ce qu'on prépare une phase huileuse à partir d'au moins les ingrédients suivants :

- une fraction huileuse, comprenant des huiles paraffiniques, linéaires ou ramifiées, ayant 10 à 30 atomes de carbone dans la chaîne, en une quantité de 5 à 50% en poids, sur la base du poids de la composition complète, ayant une plage d'ébullition comprise entre 100 et 500 °C à la pression atmosphérique et une viscosité d'au moins 35 $mm^2/s$ (cSt) à 25 °C, les huiles paraffiniques pouvant facultativement être mélangées avec des esters d'acides gras naturels de degré élevé facultativement insaturés, et d'alcools aliphatiques naturels de degré élevé facultativement insaturés, d'au moins 20 atomes de carbone dans la chaîne, en une quantité pouvant atteindre 6% en poids, sur la base du poids de la composition complète,

- un système émulsifiant, comprenant principalement :

  a) des mono- et/ou des diglycérides d'acides gras naturels de degré élevé insaturés et/ou saturés, en une quantité de l à 10% en poids, sur la base du poids de la composition complète, et

  b) des triglycérides éthoxylés estérifiés avec des acides gras selon la formule générale :

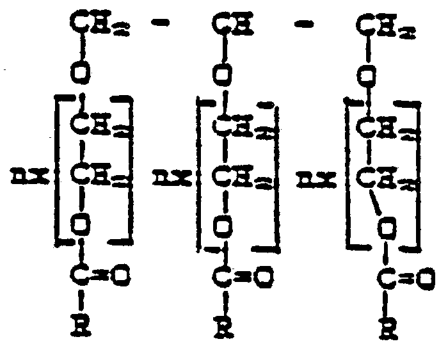

  dans laquelle n est un entier de 5 à 20 et R représente un résidu d'acides gras saturés ou insaturés, dérivés d'huiles végétales ou animales, R représentant des résidus d'acides gras identiques ou différents dans une molécule, d'où il résulte que le rapport entre la quantité de mono- et/ou de diglycérides d'une part, et de triglycérides éthoxylés d'autre part, peut varier de 10 à 100 parties de mono- et/ou de diglycérides pour l partie de triglycérides éthoxylés, la quantité complète du système émulsifiant, sur la base du poids de la composition complète, pouvant varier de 1 à 10% en poids,

- un stabilisant, constitué par des montmorillonites, dont les sites d'oxygène libre sont occupés par des groupes quaternaires, en une quantité de 0,2 à 4% en poids, sur la base du poids de la composition complète,
- un conservateur , étant identique ou différent pour la phase huileuse continue et la phase aqueuse dispersée formées, en une quantité de 0,05 à 1% en poids, sur la base du poids de la composition complète, en agitant et en chauffant jusqu' à gélification, après addition facultative d'alcanol en une quantité de 0,1 à 1% en poids, sur la base du poids de la composition complète, après quoi on refroidit à au plus 30°C et on ajoute une phase aqueuse, préparée à partir d'au moins les ingrédients suivants :
- panthénol et tyrosine ou un composé dont on peut aisément former de la tyrosine in situ dans la phase aqueuse, en un rapport molaire mutuel de tyrosine:panthénol compris entre 2:1 et 1:3, les quantités de panthénol-et de tyrosine ou de moyens procurant la tyrosine pouvant être comprises entre 1 et 5% en poids et entre 0,5 et 5% en poids respectivement, sur la base du poids de la composition complète,
- un agent causant une alcalinité pour ajuster le pH de la phase aqueuse dans la plage de 4 à 7,
- de l'eau, pour compléter à 100% en poids, après quoi on agite et on homogénéise jusqu'à l'obtention d'une dimension de particules moyennes inférieure à 3 µm.

**2.** Procédé selon la revendication 1, caractérisé en ce qu'on peut en outre ajouter les ingrédients suivants à la phase aqueuse :
- glycérol, en une quantité de 0,5 à 5% en poids, sur la base du poids de la composition complète,
- lactate de sodium et/ou citrate de sodium, en une quantité de 0,1 à 5% en poids,
- agent formateur de gel, en une quantité de 0,1 à 5% en poids.

**3.** Procédé selon les revendications 1 et 2, caractérisé en ce qu'on ajoute des ingrédients additionnels, comprenant:
- de la vitamine E, en une quantité de 0,05 à 5% en poids, et/ou
- un parfum, en une quantité de 0,1 à 0,5% en poids,

- un anti-oxydant, en une quantité de 0,01 à 3% en poids, sur la base du poids de la composition complète.

4. Procédé selon la revendication 1, caractérisé en ce qu'on prépare une composition contenant une fraction huileuse en une quantité de 10 à 40% en poids.

5. Procédé selon la revendication 1, caractérisé en ce qu'on prépare une composition, contenant une fraction huileuse mélangée avec de l'oléate d'oléyle ou du décalate d'oléyle en une quantité de 2 à 5% en poids, sur la base du poids de la composition complète.

6. Procédé selon la revendication 1, caractérisé en ce que le système émulsifiant comprend des triglycérides éthoxylés, dérivés de l'acide linoléique, l'acide oléique, l'acide linolénique ou leurs mélanges, mélangés avec des mono- et/ou des diglycérides dérivés de l'acide linoléique, l'acide linolénique, l'acide oléique, l'acide palmitique, l'acide laurique, l'acide myristique, l'acide stéarique, l'acide éléostéarique et leurs mélanges.

7. Procédé selon la revendication 1, caractérisé en ce que le rapport entre les mono- et/ou les diglycérides et les triglycérides éthoxylés est 25 parties de mono- et/ou de diglycérides pour 1 partie de triglycérides éthoxylés.

8. Procédé selon la revendication 1, caractérisé en ce qu'on utilise comme conservateur dans la phase huileuse un ou plusieurs esters d'acides p-hydroxybenzoîque en une quantité de 0,2 à 0,4% en poids.

9. Procédé selon l'une des revendications 1 à 8, caractérisé en ce qu'on utilise une fraction huileuse comprenant des huiles paraffiniques linéaires ou ramifiées ayant l2 à 25 atomes de carbone dans la chaîne.

10. Procédé selon l'une des revendications l à 9, caractérisé en ce qu ' on utilise une fraction huileuse comprenant dès huiles paraffiniques linéaires ou ramifiées en une quantité de 10 à 40% en poids.

11. Procédé selon l'une des revendications 1 a 10 caractérisé en ce que, dans le système émulsifiant, on utilise un triglycéride éthoxylé estérifié de la formule générale indiquée dans la revendication 1, dans laquelle n est un nombre entier de 7 à 15.

12. Procédé selon l'une des revendications 1 à 11, caractérisé en ce que, dans le système émulsifiant, on utilise un triglycéride éthoxylé estérifié de la formule générale indiquée dans la revendication 1, dans laquelle R est un résidu d'acides gras insaturés.

13. Procédé selon l'une des revendications 1 à 12, caractérisé en ce que, dans le système émulsifiant, on utilise un triglycéride éthoxylé estérifié de la formule générale indiquée dans la revendication 1, dans laquelle les résidus R représentent les résidus des mêmes acides gras dans une molécule.

14. Procédé selon l'une des revendications 1 à 13, caractérisé en ce qu'on utilise le panthénol sous la forme de D-panthénol.

15. Procédé selon l'une des revendications 1 à 14, caractérisé en ce qu'on utilise la tyrosine sous la forme de L-tyrosine.

16. Procédé selon l'une des revendications 1 à 15, caractérisé en ce qu'on utilise l'agent causant l'alcalinité pour ajuter le pH de la phase aqueuse dans la plage de 4 à 6.

17. Procédé selon l'une des revendications 1 à 16, caractérisé en ce qu'on utilise l'agent causant l'alcalinité sous la forme de citrate de sodium et/ou d'hydroxyde de sodium.

18. Procédé selon la revendication 2, caractérisé en ce qu'on utilise du glycérol en une quantité de 1 à 3% en poids, sur la base du poids de la composition complète.

## Ansprüche

1. Hautbräunungszusammensetzung, dadurch gekennzeichnet, daß sie wenigstens die folgenden Bestandteile enthält:
   - eine Ölfraktion mit gerad- oder verzweigtkettigen Paraffinölen mit 10 bis 30 Kohlenstoffatomen in der Kette in einer Menge von 5 bis 50 Gew.-%, bezogen auf das Gewicht der gesamten Zusammensetzung, mit einem Siedebereich zwischen 100 und 500 °C bei Atmosphärendruck und einer Viskosität von höchstens 35 $mm^2/s$ (cSt) bei 25°C, worin die Paraffinöle ggf. mit Estern von ggf. ungesättigten höheren natürlichen Fettsäuren und von ggf. ungesättigten höheren natürlichen aliphatischen Alkoholen mit höchstens 20 Kohlenstoffatomen in der Kette in einer Menge von bis zu 6 Gew.-%, bezogen auf das Gewicht der gesamten Zusammensetzung, gemischt sein können,
   - ein Emulgierungssystem, hauptsächlich enthaltend:

     a) Mono- und/oder Diglyceride von höheren ungesättigten und/oder gesättigten natürlichen Fettsäuren in einer Menge von 1 bis 10 Gew.-%, bezogen auf das Gewicht der gesamten Zusammensetzung, und

     b) ethorylierte Triglyceride, verestert mit Fettsäuren, gemäß der allgemeinen Formel

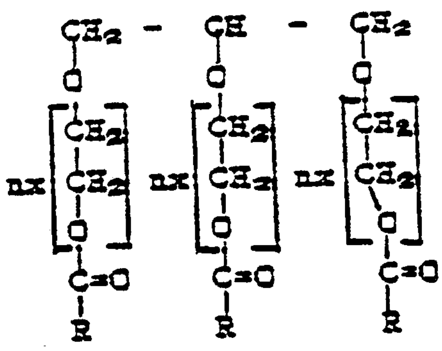

     in der n eine ganze Zahl von 5 bis 20 und R einen gesättigten oder ungesättigten Fettsäurerest darstellt, der aus pflanzlichen oder tierischen Ölen abstammt, worin R gleiche oder verschiedene Fettsäurereste in einem Molekül darstellt, wobei das Verhältnis zwischen der Menge der Mono- und/oder Diglyceride einerseits und der ethoxylierten Triglyceride andererseits von 10 bis 100 Teilen Monound/oder Diglyceride pro Teil ethoxylierte Triglyceride variieren kann, wobei die gesamte Menge des Fmulgierungssystems, bezogen auf das Gewicht der gesamten Zusammensetzung, von 1 bis 10 Gew.-% variieren kann,
   - Panthenol und Tyrosin oder eine Verbindung, aus der Tyrosin in situ in der wäßrigen Phase leicht gebildet werden kann, in einem geeigneten Molverhältnis von Tyrosin zu Panthenol zwischen 2 : 1 und 1 : 3, wobei die Mengen an Panthenol und Tyrosin oder an Tyrosin zur Verfügung stellendem Mittel 1 bis 5 Gew.-% bzw. 0,5 bis 5 Gew.-%, bezogen auf das Gewicht der gesamten Zusammensetzung, betragen können,
   - ein Alkalinität verursachendes Mittel zur Einstellung des pH-Wertes in einem Bereich von 4 bis 7,
   - einen Stabilisator, bestehend aus Montmorilloniten, deren freie Sauerstoffstellen durch quartäre Reste in einer Menge von 0,2 bis 4 Gew.-%, bezogen auf das Gewicht der gesamten Zusammensetzung, besetzt sind,
   - ein Konservierungsmittel, das für die gebildete kontinuierliche Ölphase und die disperse wäßrige Phase gleich oder verschieden ist, in einer Menge von 0,05 bis 1 Gew.-%, bezogen auf das Gewicht der gesamten Zusammensetzung,
   - Wasser ad 100 Gew.-%.

2. Zusammensetzung nach Anspruch 1, dadurch gekennzeichnet, daß sie zusätzlich zu den angegebenen Bestandteilen mindestens einen der folgenden Bestandteile enthält:
   - Vitamin E in einer Menge von 0,05 bis 5 Gew.-%,
   - Glycerin in einer Menge von 0,5 bis 5,0 Gew.-%,
   - Alkanol in einer Menge von 0,1 bis 1,0 Gew.-%,
   - Natriumlactat in einer Menge von 0,1 bis 5 Gew.-%,
   - ein gelbildendes Mittel in einer Menge von 0,1 bis 5 Gew.-%,

- einen Duftstoff in einer Menge von 0,1 bis 0,5 Gew.-%,
- Antioxydantien in einer Menge von 0,01 bis 3 Gew.-%, bezogen auf das Gewicht der gesamten Zusammensetzung.

3. Zusammensetzung nach Anspruch 1, dadurch gekennzeichnet, daß sie eine Ölfraktion in einer Menge von 10 bis 40 Gew.-% enthält.

4. Zusammensetzung nach Anspruch 1, dadurch gekennzeichnet, daß sie eine mit Oleyloleat oder Oleyldecalat in einer Menge von 2 bis 5 Gew.-%, bezogen auf das Gewicht der gesamten Zusammensetzung, gemischte Ölfraktion enthält.

5. Zusammensetzung Anspruch 1, dadurch gekennzeichnet, daß das Emulgierungssystem aus ethoxylierten Triglyceriden, die von Linolsäure, Ölsäure, Linolensäure oder Gemischen davon abstammen, gemischt mit Mono- und/oder Diglyceriden, die von Linolsäure, Linolensäure, Ölsäure, Palmitinsäure, Laurinsäure, Myristinsäure, Stearinsäure, Eleostearinsäure und Gemischen davon abstammen, zusammengesetzt ist.

6. Zusammensetzung nach Anspruch 1, dadurch gekennzeichnet, daß das Verhältnis zwischen Mono- und/oder Diglyceriden und ethoxylierten Triglyceriden 25 Teile Mono- und/oder Diglyceride pro Teil ethoxylierte Triglyceride beträgt.

7. Zusammensetzung nach Anspruch 1, dadurch gekennzeichnet, daß als Konservierungsmittel in der Ölphase ein oder mehrere Ester der p-Hydroxybenzoesäure in einer Menge von 0,2 bis 0,4 Gew.-% vorliegen.

8. Zusammensetzung nach einem der Ansprüche 1 bis 7, dadurch gekennzeichnet, daß die Ölfraktion gerad- oder verzweigtkettige Paraffinöle mit 12 bis 25 Kohlenstoffatomen in der Kette enthält.

9. Zusammensetzung nach einem der Ansprüche 1 bis 8, dadurch gekennzeichnet, daß die Ölfraktion gerad- oder verzweigtkettige Paraffinöle in einer Menge von 10 bis 40 Gew.-% enthält.

10. Zusammensetzung nach einem der Ansprüche 1 bis 9, dadurch gekennzeichnet, daß in der allgemeinen Formel der veresterten ethoxylierten Triglyceride des Emulgierungssystems, wie in Anspruch 1 angegeben, n eine ganze Zahl von 7 bis 15 darstellt.

11. Zusammensetzung nach einem der Ansprüche 1 bis 10, dadurch gekennzeichnet, daß in der allgemeinen Formel der veresterten ethoxylierten Triglyceride des Emulgierungssystems, wie in Anspruch 1 angegeben, R einen ungesättigten Fettsäurerest darstellt.

12. Zusammensetzung nach einem der Ansprüche 1 bis 11, dadurch gekennzeichnet, daß in der allgemeinen Formel der veresterten ethoxylierten Triglyceride des Emulgierungssystems, wie in Anspruch 1 angegeben, die Reste R die gleichen Fettsäurereste in einem Molekül darstellen.

13. Zusammensetzung nach einem der Ansprüche 1 bis 12, dadurch gekennzeichnet, daß sie Panthenol in Form von DPanthenol enthält.

14. Zusammensetzung nach einem der Ansprüche 1 bis 13, dadurch gekennzeichnet, daß sie Tyrosin in Form von L-Nrosin enthält.

15. Zusammensetzung nach einem der Ansprüche 1 bis 14, dadurch gekennzeichnet, daß sie das Alkalinität verursachende Mittel enthält, um den pH-Wert der wäßrigen Phase im Bereich von 4 bis 6 einzustellen.

16. Zusammensetzung nach einem der Ansprüche 1 bis 15, dadurch gekennzeichnet, daß sie das Alkalinität verursachende Mittel in Form von Natriumcitrat und/oder Natriumhydroxid enthält.

17. Zusammensetzung nach Anspruch 2, dadurch gekennzeichnet, daß sie Natriumlactat in einer Menge von 0,5 bis 2 Gew.-% enthält.

18. Verfahren zur Herstellung einer Zusammensetzung gemäß einem der Ansprüche 1 bis 17, dadurch gekennzeichnet, daß eine ölige Phase durch Zugabe des Emulgierungssystems, des Konservierungsmittels und des Stabilisators zu den öligen Bestandteilen unter Rühren und Erwärmen bis zur Gelbildung nach Zugabe von Alkohol hergestellt wird, gefolgt von einer Abkühlung auf höchstens 30 °C und Zugabe der wäßrigen Phase, die aus Wasser, Konservierungsmittel, Glycerin, Panthenol, Tyrosin oder der das Tyrosin zur Verfügung stellenden Verbindung, Alkalinität verursachendem Mittel, soweit notwendig, und Carrageenat zusammengesetzt ist.

19. Verfahren zur Herstellung einer Hautbräunungszusammensetzung, dadurch gekennzeichnet, daß eine ölige Phase aus wenigstens den folgenden Bestandteilen, nämlich
- einer Ölfraktion gemäß einem der Ansprüche 1, 8 oder 9,
- einem Emulgierungssystem gemäß einem der Ansprüche 1, 10, 11 oder 12,
- einem Stabilisator gemäß Anspruch 1 und
- einem Konservierungsmittel gemäß Anspruch 1, unter Rühren und Erhitzen bis zur Gelbildung, ggf. nach Zugabe von Alkanol in einer Menge von 0,1 bis 1,0 Gew.-%, bezogen auf das Gewicht der gesamten Zusammensetzung, gefolgt von einer Abkühlung auf höchstens 300 °C, hergestellt und eine wäßrige Phase zugegeben wird, die aus wenigstens den folgenden Bestandteilen
- Panthenol und
  Tyrosin oder einer Verbindung, aus der in situ in der wäßrigen Phase leicht Tyrosin gebildet wird, in einem Molverhältnis von Tyrosin zu Panthenol zwischen 2 : 1 und 1 : 3, wobei die Menge von Panthenol und Tyrosin oder Tyrosin zur Verfügung stellendem Mittel 1 bis 5 Gew.-% bzw. 0,5 bis 5 Gew.-%, bezogen auf das Gewicht der gesamten Zusammensetzung, betragen können,
- Alkalinität verursachendes Mittel zur Einstellung des pH-Wertes der wäßrigen Phase in einem Bereich von 4 bis 7 und
- Wasser ad 100 Gew.-% hergestellt worden ist, gefolgt von Rühren und Homogenisieren, bis eine durchschnittliche Teilchengröße von < 3 u erreicht ist.

20. Verfahren nach Anspruch 19, dadurch gekennzeichnet, daß Panthenol in Form von D-Panthenol, oder Tyrosin in Form von L-Tyrosin, oder das Alkalinität verursachende Mittel zur Einstellung des pH-Werts der wäßrigen Phase im Bereich von 4 bis 6, oder das Alkalinität verursachende Mittel in Form von Natriumcitrat und/oder Natriumhydroxid verwendet wird.

21. Verfahren nach Anspruch 20, dadurch gekennzeichnet, daß die folgenden Bestandteile zusätzlich zu der wäßrigen Phase gegeben werden können:
- Glycerin in einer Menge von 0,5 bis 5,0 Gew.-%, bezogen auf das Gewicht der gesamten Zusammensetzung,
- Natriumlactat und/oder Natriumcitrat in einer Menge von 0,1 bis 5 Gew.-%,
- ein gelbildendes Mittel in einer Menge von 0,1 bis 5 Gew.-%.

22. Verfahren nach Anspruch 21, dadurch gekennzeichnet, daß Glycerin in einer Menge von 1 bis 3 Gew.-%, bezogen auf das Gewicht der gesamten Zusammensetzung, verwendet wird.

23. Verfahren nach einem der Ansprüche 19 bis 22, dadurch gekennzeichnet, daß zusätzliche Bestandteile zugegeben werden, bestehend aus
- Vitamin E in einer Menge von 0,05 bis 5 Gew.-% und/oder
- einem Duftstoff in einer Menge von 0,1 bis 0,5 Gew.-%,
- einem Antioxidans in einer Menge von 0,01 bis 3 Gew.-%, bezogen auf das Gewicht der gesamten Zusammensetzung.

24. Verfahren nach Anspruch 19, dadurch gekennzeichnet, daß eine Zusammensetzung hergestellt wird, die eine Ölfraktion in einer Menge von 10 bis 40 Gew.-% enthält.

25. Verfahren nach Anspruch 19, dadurch gekennzeichnet, daß eine Zusammensetzung hergestellt wird, die eine Ölfraktion, gemischt mit Oleyloleat oder Oleyldecalat in einer Menge von 2 bis 5 Gew.-%, bezogen auf das Gewicht der gesamten Zusammensetzung, enthält.

26. Verfahren nach Anspruch 19, dadurch gekennzeichnet, daß das Emulgierungssystem aus ethoxylierten Triglyceriden, die von Linolsäure, Ölsäure, Linolinsäure oder Gemischen davon abstammen, gemischt

mit Mono- und/oder Diglyceriden, die von Linolsäure, Linolinsäure, Ölsäure, Palmitinsäure, Laurinsäure, Myristinsäure, Stearinsäure, Eleostearinsäure und Gemischen davon abstammen, zusammengesetzt ist.

**27.** Verfahren nach Anspruch 19, dadurch gekennzeichnet, daß das Verhältnis zwischen Mono- und/oder Diglyceriden und ethoxylierten Triglyceriden 25 Teile Mono- und/oder Diglyceride pro Teil ethoxylierte Triglyceride beträgt.

**28.** Verfahren nach Anspruch 19, dadurch gekennzeichnet, daß als Konservierungsmittel in der öligen Phase ein oder mehrere Ester der p-Hydroxybenzoesäure in einer Menge von 0,2 bis 0,4 Gew.-% verwendet werden.

Patentansprüche für folgende Vertragsstaaten: AT ES GR:

**1.** Verfahren zur Herstellung einer Hautbräunungszusammensetzung, dadurch gekennzeichnet, daß eine ölige Phase aus wenigstens den folgenden Bestandteilen, nämlich

- einer Ölfraktion mit gerad- oder verzweigtkettigen Paraffinölen mit 10 bis 30 Kohlenstoffatomen in der Kette in einer Menge von 5 bis 50 Gew.-%, bezogen auf das Gewicht der gesamten Zusammensetzung, mit einem Siedebereich zwischen 100 und 500° C bei Atmosphärendruck und einer Viskosität von höchstens 35 $mm^2/s$ (cSt) bei 25° C, worin die Paraffinöle ggf. mit Estern von ggf. ungesättigten höheren natürlichen Fettsäuren und von ggf. ungesättigten höheren natürlichen aliphatischen Alkoholen mit höchstens 20 Kohlenstoffatomen in der Kette in einer Menge von bis zu 6 Gew.-%, bezogen auf das Gewicht der gesamten Zusammensetzung, gemischt sein können,
- einem Emulgierungssystem, hauptsächlich enthaltend:
  a) Mono- und/oder Diglyceride von höheren ungesättigten und/oder gesättigten natürlichen Fettsäuren in einer Menge von 1 bis 10 Gew.-%, bezogen auf das Gewicht der gesamten Zusammensetzung, und
  b) ethoxylierte Triglyceride, verestert mit Fettsäuren, gemäß der allgemeinen Formel

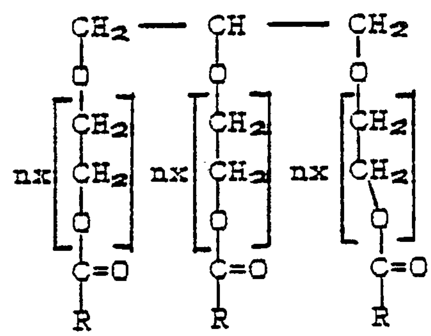

  in der n eine ganze Zahl von 5 bis 20 und R einen gesättigten oder ungesättigten Fettsäurerest darstellt, der aus pflanzlichen oder tierischen Ölen abstammt, worin R gleiche oder verschiedene Fettsäurereste in einem Molekül darstellt, wobei das Verhältnis zwischen der Menge der Mono- und/oder Diglyceride einerseits und der ethoxylierten Triglyceride andererseits von 10 bis 100 Teilen Monound/oder Diglyceride pro Teil ethoxylierte Triglyceride variieren kann, wobei die gesamte Menge des Emulgierungssystems, bezogen auf das Gewicht der gesamten Zusammensetzung, von 1 bis 10 Gew.-% variieren kann,
- einem Stabilisator, bestehend aus Montmorilloniten, deren freie Sauerstoffstellen durch quartäre Reste in einer Menge von 0,2 bis 4 Gew.-%, bezogen auf das Gewicht der gesamten Zusammensetzung, besetzt sind,
- einem Konservierungsmittel, das für die gebildete kontinuierliche ölige Phase und die disperse wäßrige Phase gleich oder verschieden ist, in einer Menge von 0,05 bis 1 Gew.-%, bezogen auf die gesamte Zusammensetzung, unter Rühren und Erhitzen bis zur Gelbildung, ggf. nach Zugabe von Alkanol in einer Menge von 0,1 bis 1,0 Gew.-%, bezogen auf das Gewicht der gesamten Zusammensetzung, gefolgt von einer Abkühlung auf höchstens 30°C, hergestellt und eine wäßrige Phase zugegeben wird, die aus wenigstens den folgenden Bestandteilen
- Panthenol und
  Tyrosin oder einer Verbindung, aus der in situ in der wäßrigen Phase leicht Tyrosin gebildet wird,

in einem Molverhältnis von Tyrosin zu Panthenol zwischen 2 : 1 und 1 : 3, wobei die Mengen von Panthenol und Tyrosin oder an Tyrosin zur Verfügung stellendem Mittel 1 bis 5 Gew.-% bzw. 0,5 bis 5 Gew.-%, bezogen auf das Gewicht der gesamten Zusammensetzung, betragen können,
- Alkalinität verursachendes Mittel zur Einstellung des pH-Wertes der wäßrigen Phase in einem Bereich von 4 bis 7 und
- Wasser ad 100 Gew.-% hergestellt worden ist, gefolgt von Rühren und Homogenisieren, bis eine durchschnittliche Teilchengröße von < 3 μ erreicht ist.

2. Verfahren nach Anspruch 1, dadurch gekennzeichnet, daß die folgenden Bestandteile zusätzlich zu der wäßrigen Phase hinzugefügt werden können:
   - Glycerin in einer Menge von 0,5 bis 5,0 Gew.-%, bezogen auf das Gewicht der gesamten Zusammensetzung,
   - Natriumlactat und/oder Natriumcitrat in einer Menge von 0,1 bis 5 Gew.-%,
   - ein gelbildendes Mittel in einer Menge von 0,1 bis 5 Gew.-%.

3. Verfahren nach Anspruch 1 oder 2, dadurch gekennzeichnet, daß zusätzliche Bestandteile zugegeben werden, bestehend aus
   - Vitamin E in einer Menge von 0,05 bis 5 Gew.-% und/oder
   - ein Duftstoff in einer Menge von 0,1 bis 0,5 Gew.-%,
   - ein Antioxidans in einer Menge von 0,01 bis 3 Gew.-%, bezogen auf das Gewicht der gesamten Zusammensetzung.

4. Verfahren nach Anspruch 1, dadurch gekennzeichnet, daß eine Zusammensetzung hergestellt wird, die eine Ölfraktion in einer Menge von 10 bis 40 Gew.-% enthält.

5. Verfahren nach Anspruch 1, dadurch gekennzeichnet, daß eine Zusammensetzung hergestellt wird, die eine Ölfraktion gemischt mit Oleyloleat oder Oleyldecalat in einer Menge von 2 bis 5 Gew.-%, bezogen auf das Gewicht der gesamten Zusammensetzung, enthält.

6. Verfahren nach Anspruch 1, dadurch gekennzeichnet, daß das Emulgierungssystem aus ethoxylierten Triglyceriden, die von Linolsäure, Ölsäure, Linolensäure oder Gemischen davon abstammen, gemischt mit Mono- und/oder Diglyceriden, die von Linolsäure, Linolensäure, Ölsäure, Palmitinsäure, Laurinsäure, Myristinsäure, Stearinsäure, Eleostearinsäure und Gemischen davon abstammen, zusammengesetzt ist.

7. Verfahren nach Anspruch 1, dadurch gekennzeichnet, daß das Verhältnis zwischen Mono- und/oder Diglyceriden und ethoxylierten Triglyceriden 25 Teile Mono- und/oder Diglyceride pro Teil ethoxylierte Triglyceride beträgt.

8. Verfahren nach Anspruch 1, dadurch gekennzeichnet, daß als Konservierungsmittel in der öligen Phase ein oder mehrere Ester der p-Hydroxybenzoesäure in einer Menge von 0,2 bis 0,4 Gew.-% verwendet werden.

9. Verfahren nach einem der Ansprüche 1 bis 8, dadurch gekennzeichnet, daß eine Ölfraktion, enthaltend geradoder verzweigtkettige Paraffinöle mit 12 bis 15 Kohlenstoffatomen in der Kette, verwendet wird.

10. Verfahren nach einem der Ansprüche 1 bis 9, dadurch gekennzeichnet, daß eine Ölfraktion, enthaltend geradoder verzweigtkettige Paraffinöle in einer Menge von 10 bis 40 Gew.-%, verwendet wird.

11. Verfahren nach einem der Ansprüche 1 bis 10, dadurch gekennzeichnet, daß in dem Emulgierungssystem ein verestertes ethoxyliertes Triglycerid der allgemeinen Formel, wie in Anspruch 1 angegeben, verwendet wird, worin n eine ganze Zahl von 7 bis 15 darstellt.

12. Verfahren nach einem der Ansprüche 1 bis 11, dadurch gekennzeichnet, daß als Emulierungssystem ein verestertes ethoxyliertes Triglyceride der allgemeinen Formel, wie in Anspruch 1 angegeben, verwendet wird, worin R einen ungesättigten Fettsäurerest darstellt.

13. Verfahren nach einem der Ansprüche 1 bis 12, dadurch gekennzeichnet, daß in dem Emulgierungssystem ein verestertes ethoxyliertes Triglycerid der allgemeinen Formel, wie in Anspruch 1 angegeben, verwendet wird, worin die Reste R die gleichen Fettsäurereste in einem Molekül darstellen.

**14.** Verfahren nach einem der Ansprüche 1 bis 13, dadurch gekennzeichnet, daß Panthenol in Form von D-Panthenol verwendet wird.

**15.** Verfahren nach einem der Ansprüche 1 bis 14, dadurch gekennzeichnet, daß Tyrosin in Form von L-Tyrosin verwendet wird.

**16.** Verfahren nach einem der Ansprüche 1 bis 15, dadurch gekennzeichnet, daß das Alkalinität verursachende Mittel verwendet wird, um den pH-wert der wäßrigen Phase im Bereich von 4 bis 6 einzustellen.

**17.** Verfahren nach einem der Ansprüche 1 bis 16, dadurch gekennzeichnet, daß das Alkalinität verursachende Mittel in Form von Natriumcitrat und/oder Natriumhydroxid verwendet wird.

**18.** Verfahren nach Anspruch 2, dadurch gekennzeichnet, daß Glycerin in einer Menge von 1 bis 3 Gew.` %, bezogen auf das Gewicht der gesamten Zusammensetzung, verwendet wird.